(19) Europäisches Patentamt
European Patent Office
Office européen des brevets

(11) EP 1 537 130 B1

(12) # EUROPÄISCHE PATENTSCHRIFT

(45) Veröffentlichungstag und Bekanntmachung des Hinweises auf die Patenterteilung:
**23.11.2011 Patentblatt 2011/47**

(21) Anmeldenummer: **03757825.9**

(22) Anmeldetag: **11.09.2003**

(51) Int Cl.:
***C07F 9/40*** (2006.01) ***C07F 9/46*** (2006.01)
***C07F 9/48*** (2006.01) ***C07F 9/50*** (2006.01)

(86) Internationale Anmeldenummer:
**PCT/EP2003/010131**

(87) Internationale Veröffentlichungsnummer:
**WO 2004/026884 (01.04.2004 Gazette 2004/14)**

(54) **PHOSPHORGRUPPENHALTIGE CARBONSÄUREDERIVATE MIT ORGANISCH POLYMERISIERBAREN GRUPPEN**

CARBOXYLIC ACID DERIVATIVES CONTAINING PHOSPHOROUS GROUPS AND ORGANICALLY POLYMERISABLE GROUPS

DERIVES D'ACIDE CARBOXYLIQUE CONTENANT DES GROUPES PHOSPHORES ET PRESENTANT DES GROUPES ORGANIQUEMENT POLYMERISABLES

(84) Benannte Vertragsstaaten:
**AT BE BG CH CY CZ DE DK EE ES FI FR GB GR HU IE IT LI LU MC NL PT RO SE SI SK TR**

(30) Priorität: **11.09.2002 DE 10242106**

(43) Veröffentlichungstag der Anmeldung:
**08.06.2005 Patentblatt 2005/23**

(73) Patentinhaber: **Fraunhofer-Gesellschaft zur Förderung der angewandten Forschung e.V.
80686 München (DE)**

(72) Erfinder:
- **WOLTER, Herbert
97941 Tauberbischofsheim (DE)**
- **NIQUE, Somchith
97249 Eisingen (DE)**

(56) Entgegenhaltungen:
**EP-A- 0 667 364 WO-A-00/58316
WO-A-01/74826 WO-A-02/088222
DE-A- 1 495 383 FR-A- 2 767 829
US-A- 3 762 865 US-A- 3 839 207
US-A- 4 044 075**

- **DATABASE CA [Online] CHEMICAL ABSTRACTS SERVICE, COLUMBUS, OHIO, US; KATO, EIICHI: "Oil-based inks for electrostatic ink-jet printing producing images with good clarity and high strength and freedom from nozzle clogging" retrieved from STN Database accession no. 138: 370460 XP002266660 & JP 2003 138183 A (FUJI PHOTO FILM CO., LTD., JAPAN) 14. Mai 2003 (2003-05-14)**
- **DATABASE CROSSFIRE BEILSTEIN [Online] Beilstein Institut zur Förderung der Chemischen Wissenschaften, Frankfurt am Main, DE; Database accession no. BRN 7176797; BRN 7175561 XP002266661 & HEWITT, D. G. ET AL.: AUST. J. CHEM., Nr. 37, 1984, Seiten 1631-1642,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft neue Carbonsäurederivate, die mindestens eine organisch polymerisierbare Gruppe und mindestens eine reaktionsfähige oder die Eigenschaften des Moleküls modulierende phosphorhaltige Gruppe aufweisen. Weiterhin betrifft die Erfindung Verfahren zum Herstellen der erfindungsgemäßen Moleküle und ihre Verwendung im Dentalbereich und zur Beeinflussung der Eigenschaften von Materialien.

Es ist eine Vielzahl von organisch substituierten, ggf. auch C=C-Doppelbindungen enthaltenden Phosphorverbindungen bekannt, die sich in organische Polymere einbauen oder direkt polymerisieren lassen. Das Vorhandensein von phosphorhaltigen Gruppen in Polymeren kann eine Reihe von Eigenschaften dieser Verbindungen positiv beeinflussen, z.B. eine Steigerung der thermischen Stabilität, eine Verminderung der Entflammbarkeit, eine Verbesserung der Hafteigenschaften und/oder eine Verbesserung der Löslichkeit in polaren Solventien bewirken. Doppelbindungshaltige Phosphorverbindungen (z.B. entsprechend substituierte Phosphonsäurederivate) werden insbesondere auch im Dentalbereich eingesetzt, wobei sie z.B. aufgrund der Komplexierung von $Ca^{2+}$-Ionen sehr effektive Haftvermittler/Bondings darstellen.

Allerdings besitzen die bekannten polymerisierbaren Phosphorverbindungen Nachteile. So sind diejenigen Verbindungen, deren Phosphoratom(e) über Sauerstoff mit der organischen Komponente des Moleküls verbunden ist/sind, hydrolytisch instabil. Beispielsweise offenbart die DE 27 11 234 C3 von Kuraray Co. Phosphorsäurediester, deren beide Estergruppen in wäßrigem Milieu jeweils verseift werden, sowie Phosphonsäureester, die immer noch eine hydrolysierbare Gruppe aufweisen. Die EP 0554890 von Dentspley offenbart phosphorsäurederivatisierte Poly(meth)acrylate als Basis für lichthärtbare Glasionomerzemente, die ebenfalls hydrolyseempfindlich sind. Für den Dentalbereich geeignete Materialien sollten diese Eigenschaft jedoch nicht besitzen, da sie im Mund immer einer Umgebung mit hohem Feuchtegehalt ausgesetzt sind. Zwar gibt es auch eine Reihe von doppelbindungshaltigen Phosphorverbindungen, in denen die oder eine organische Gruppe direkt an den Phosphor gebunden vorliegt. Diese sind jedoch nur sehr aufwendig herstellbar. So beschreibt die DE 199 18 974 A1 Dentalwerkstoffe mit polymerisierbaren Phosphonsäuren, die sich sehr gut als Zahnfüllmaterialien eignen, da sie eine starke und dauerhafte Anbindung des Füllkörpers an den Zahnuntergrund gewährleisten. Bei diesen Dentalwerkstoffen handelt es sich um (Meth-)Acrylsäurephosphonsäurealkylester, die auf folgende Weise zugänglich sind: Phosphorsäuredialkylester werden mit doppelbindungshaltigen Ethern zu Monoalkylphosphonsäureestern umgesetzt, die, ggf. nach Hydrolyse, mit (Meth-)-Acrylsäurechlorid zur Reaktion gebracht werden. Das Produkt wird anschließen einer Hydrolyse unterworfen. Die Herstellung der hydrolysestabilen und polymerisierbaren Acrylphosphonsäure der DE 197 46 708 C2 erfordert die Synthese von Halogenmethylacrylsäureestern sowie von geschützten mono- oder difunktionellen Phosphonsäureestem, also von Ausgangsverbindungen, die jeweils nur sehr mühsam und aufwendig darzustellen sind.

Phosphorsäureester und Phosphonsäureester, die jeweils eine (Meth-)Acrylatgruppe enthalten könne, werden in WO 01/74826 A1 zur Herstellung von phosphorhaltigen Polyurethan-Copolyeren vorgeschlagen, die ihrerseits als Flammverzögerer, Korrosionshemmer, Dispersionmittel für Pigmente und/oder Haftungsverbesserer eingesetzt werden können.

US 3,762,865 betrifft ein Verfahren zum Flammfestmachen von Textilien, Papier oder dergleichen, bei welchem auf dem Substrat ein Allyl-2-carbamoylalkylenphosphonat auf das flammfest zu machende Substrat aufgebracht wird. Das Phosphonat kann beispielsweise N,N'-Methylenbis-(diatlyl-2-carbamoylalkylphosphonat) sein.
US 3,839,207 betrifft Polymere dieser Phosphonate.

WO 00/58316 A betrifft Organophosphorverbindungen für polymerisierbare Dentalmaterialien, darunter vor allem Phosphatverbindungen. Die Ausbeute der zugrunde liegenden monomeren Phosphat-Monoester soll verbessert werden, und bei der Herstellung sollen weniger verfärbende Nebenprodukte anfallen. Auch die Lagerstabilität soll verbessert werden. Neben der Beschreibung dieser Verbindungen findet sich eine kurze Passage, die zwei Phosphonat-Verbindungen mit je einer Methacrylatgruppe betrifft, die über einen sehr komplexen Syntheseweg hergestellt werden. Das Dokument schweigt zur Frage der Hydrolysestabilität von Phosphat- und Phosphonatverbindungen.

Aufgabe der vorliegenden Erfindung ist es, organisch polymerisierbare, phosphorhaltige Verbindungen bereitzustellen, die auf der einen Seite im obigen Sinne hydrolysestabil und auf der anderen Seite relativ einfach zugänglich sind. Insbesondere, aber nicht nur, sollen diese Verbindungen für die Herstellung von Materialien geeignet sein, die im Dentalbereich einsetzbar sind.

Überraschend konnte gefunden werden, dass Carbonsäurederivate mit mindestens einer an den Kohlenstoff des Säureteils der Moleküle gebundenen, phosphorhaltigen Gruppe und mindestens einer organisch polymerisierbaren Gruppe im Säurederivat-Teil des Moleküls (d.h. im Ester-, Amid-, Thioester-Rest oder dgl.) die gestellte Aufgabe löst.

Erfindungsgemäß werden demzufolge Verbindungen der Formel

$$[(R^1)(R^2)P(O)\text{-}(CHR^3)\text{-}(CHR^4)\text{-}(CHR^5)_m\text{-}C(O)X\text{-}]_n\{B\} \qquad I$$

bereitgestellt, worin die Reste und Indices die folgende Bedeutung haben:

X ist O, NH, $NR^6$ oder S,
$R^1$, $R^2$ können unabhängig voneinander Wasserstoff sein; vorzugsweise sind sie ggf. substituiertes Alkyl, Alkenyl, Aryl, Alkylaryl oder Arylalkyl oder ggf. substituiertes Alkoxy, Aryloxy, Alkylaryloxy oder Arylalkyloxy oder Hydroxy mit stärker bevorzugt jeweils 1-6 Kohlenstoffatomen für offenkettige aliphatische Gruppen und stärker bevorzugt 6 bis 12 Kohlenstoffatomen für cycloaliphatische oder aromatische Gruppen, wobei die Substitution beispielsweise mit Halogen oder Aminogruppen, sauerstoffhaltigen und/oder schwefelhaltigen Resten erfolgen kann,
$R^3$, $R^4$, $R^5$ und $R^6$ sind unabhängig voneinander Wasserstoff oder ggf. substituiertes Alkyl, Alkenyl, Aryl, Alkylaryl oder Arylalkyl mit vorzugsweise jeweils 1-6 Kohlenstoffatomen für offenkettige aliphatische Gruppen und vorzugsweise 6 bis 12 Kohlenstoffatome für cycloaliphatische oder aromatische Gruppen, wobei die Substitution beispielsweise mit Halogen oder Aminogruppen, sauerstoffhaltigen und/oder schwefelhaltigen Resten erfolgen kann.
{B} ist ein geradkettiger oder verzweigter Rest, der mindestens zwei Vinyl-, Allyl-. Norbonen-, Glycidyl-, Acrylat-, Methacrylat-, Thioacrylat- oder Thiomethacrylat-Gruppen oder von (Meth)Acrylsäureamiden abgeleitete Gruppen aufweist und der kein Siliciumatom und vorzugsweise bis zu 50 Kohlenstoffatome enthält; {B} weist n Bindungen zu den n phosphorhaltigen Resten auf, deren Definition in eckige Klammern gesetzt ist;
m ist vorzugsweise 0, kann aber auch 1, 2, 3, 4 oder ggf. sogar eine höhere Zahl bis 20 sein, und
n ist vorzugsweise 1, kann aber auch 2, 3, 4 oder ggf. sogar eine höhere Zahl bis 20 sein.
{B} ist wie erwähnt ein Rest mit n Bindungen zu den n phosphorhaltigen Resten. Wenn n größer als 1 ist, enthält {B} dementsprechend mehrere Gruppen, die über X an diese Reste gebunden sind. Wenn es sich bei den organisch polymensierbare(n) Gruppe(n) des Restes {B} um Vinyl- bzw. Allylgruppe handelt, sind diese zweckmäßigerweise über eine reine Kohlenstoffkette an die den Phosphorteil des Moleküls mit dem Teil {B} verbindende Gruppe C(O)X gebunden. Besonders bevorzugt leitet sich {B} von einer substituierten oder unsubstituierten Verbindung mit Acrylat- oder Methacrylatgruppen oder den Thioanalogen hiervon oder deren Säureamiden ab. Es können gegebenenfalls sogar mehr als zwei verschiedene Gruppen, z.B. eine Acrylat- und zwei Methacrylatgruppen oder vice versa in {B} vorhanden sein. {B} kann ein durchgehendes Kohlenstoffskelett aufweisen, die Kohlenstoffkette(n) (Haupt- und/oder Seitenkette(n)) kann/können aber auch durch Heteroatome bzw. Gruppen wie O, S, SO, NH, NHCO, PR, POR, CONHCO, COO, NHCOO oder dergleichen unterbrochen sein. Das Kohlenstoffskelett kann ausschließlich aliphatisch sein, und zwar mit offenen und/oder geschlossenen Strukturen, {B} kann aber auch einen oder mehrere aromatische(n) Kem(e) oder kondensierte Systeme oder Triazingruppen oder dgl. aufweisen, z.B. Bisphenol-A-Strukturen oder dergleichen. Ferner können die Gruppen oder Strukturen beliebig substituiert sein, z.B. mit Säure-, Säureamid-, Ester oder Aminogruppen. Dagegen ist die vorliegende Erfindung nur sehr nachrangig auf Verbindungen der Formel (I) gerichtet, in denen {B} eine oder mehrere isolierte oder oligomerisierte Isoprengruppe(n) aufweist, da diese in der Regel gegenüber einer weiteren organischen Vernetzung gegenüber relativ inert sind.

Der Rest {B} ist über eine Ester-, Amid- oder Thioesterbrücke -C(O)X- mit dem die phosphorhaltige Gruppe aufweisenden Molekülteil $(R^1)(R^2)P(O)-(CHR^3)-(CHR^4)-(CHR^5)_m-$ verbunden.
Es ist bevorzugt, dass X Sauerstoff oder Stickstoff bedeutet. Besonders bevorzugt ist X Sauerstoff.
Weiterhin ist es besonders bevorzugt, dass der Rest {B} mehr als eine Acrylat-, Methacrylatgruppe oder Glycidylgruppe enthält. Die Acrylat- und/oder Methacrylatgruppen können mit einem relativ kurzkettigen (insbesondere drei bis zehn Kohlenstoffatome enthaltenden) Oligoalkohol, z.B, einem Di- oder Tri- oder Tetra- oder Pentaalkohol wie Glycol, Glycerin, Trimethylolpropan oder Pentaerythrit oder dergleichen verestert vorliegen, wobei der Alkohol vorzugsweise über eine weitere seiner Alkoholfunktionen an die C(O)X-Gruppe gebunden vorliegt. Statt der Alkoholfunktionen können in geeigneten Fällen auch Thio- oder Aminfunktionen vorhanden sein; ggf. auch gemischt mit Alkoholfunktionen. Dann kann entweder die Kopplungsgruppe CO(X) eine Säureamidgruppe oder Thioestergruppe sein, und/oder die oben genannten doppelbindungshaltigen Gruppen können über Amin- bzw. Thiofunktionen angebunden sein. In diesen Fällen enthält {B} z.B. Thio(meth)acrylat- oder (Meth)Acrylsäureamidgruppen. {B} kann die vorstehend skizzierten Oligo(meth) acrylatester, -amide, thioester enthalten oder aber auch daraus bestehen. Unter dieser Gruppe sind die Oligo(meth) acrylatester bevorzugt.
In besonders bevorzugten Ausgestaltungen der Erfindung ist eine Kombination aus mindestens zwei der in den voranstehenden Absätzen als bevorzugt oder besonders bevorzugt genannten Gruppen oder Indices verwirklicht, in stärker bevorzugten Ausgestaltungen mindestens drei, und in noch stärker bevorzugten mindestens vier. Ganz besonders bevorzugt ist, dass im letzteren Falle der Rest {B} einen, zwei, drei oder noch mehr Reste, ausgewählt unter Acrylatresten oder Methacrylatresten oder einer Mischung aus beiden, besitzt.
Weitere Ausgestaltungen der Erfindung ergeben sich aus den beigefügten Unteransprüchen.
In der DE 44 16 857 A1 sind Carbonsäure-funktionalisierte (Meth-)Acrylatalkoxysilane beschrieben, die sich

durch eine Vielfalt von Möglichkeiten auszeichnen, die Eigenschaften der daraus resultierenden anorganisch-organischen Verbundpolymere zu variieren bzw. einzustellen. Bedingt durch die enthaltenen Carbonsäuregruppen ergeben sich zusätzliche Reaktionsmöglichkeiten (z.B. Glasionomerreaktionen) sowie eine verbesserte Haftung auf anorganischen Oberflächen. Die vorliegend offenbarten Phosphorverbindungen übertreffen die haftvermittelnde Wirkung der in den dort genannten Zusammensetzungen enthaltenen Carbonsäurereste jedoch bei weitem. Daher werden mit den vorliegenden Verbindungen der Formel (I) Materialien bereitgestellt, die den dort vorgeschlagenen Substanzen überlegen sind.

Die erfindungsgemäßen Verbindungen lassen sich auf verschiedenen Wegen herstellen. Besonders günstig und bequem, außerdem auf Basis billiger Ausgangsverbindungen, gelingt ihre Herstellung durch die Umsetzung von Verbindungen der Formel (II)

$$[Y\text{-}(CHR^5)_m\ C(O)X\text{-}]_n\{B\} \qquad II$$

mit entsprechenden Phosphorverbindungen, die eine P-H-Bindung enthalten, z.B. Dialkylphosphiten $HP(O)(OR)_2$, Dialkylphosphinoxiden $HP(O)R_2$ oder gemischten Verbindungen (z.B. $R^1$ Alkyl, $R^2$ Alkoxy). Vorzugsweise erfolgt die Reaktion unter Zugabe eines radikalischen (z.B. bei nichtaktivierten Doppelbindungen) oder basischen (z.B. bei aktivierten Doppelbindungen) Katalysators, z.B. einem Natriumalkoholat oder eines tertiären Amins, z.B. einer Trialkylaminverbindung. Dabei ist Y eine organische Gruppe, z.B. eine Vinyl-, Allyl-, Norbomen-, oder Glycidylgruppe, die mit dem an das Phosphoratom gebundenen Wasserstoff bei dieser Reaktion zu einer $(CHR^3)$-$(CHR^4)$-Gruppe umgesetzt wird. Bevorzugt handelt es sich bei Y um eine Gruppe, die Teil eines Michaelsystems ist, z.B. um den Rest $CH_2$=CZ mit Z gleich H oder $CH_3$, und gleichzeitig

ist m in dieser Ausgestaltung vorzugsweise 0, d.h. Y-C(O) ist in diesem Fall ein (Meth-)Acrylatrest (wenn X gleich O ist) oder ein entsprechendes Derivat (z.B. mit X gleich S oder $NR^6$ mit $R^6$ gleich Wasserstoff, $C_1$-$C_6$-Alkyl oder $C_6$-$C_{12}$-Aryl). Sehr geeignet ist die Umsetzung von Dialkylphosphiten mit Oligo(meth)acrylaten wie Trimethylolpropan trimethacrylat (TMPT(M)A) oder Pentaerythritol-Triacrylat oder (Meth)Acrylaten der nachstehenden Formeln:

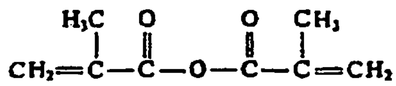

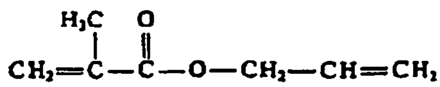

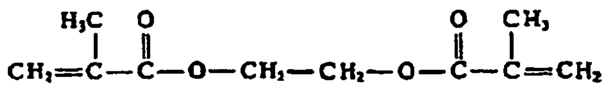

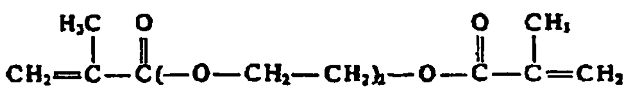

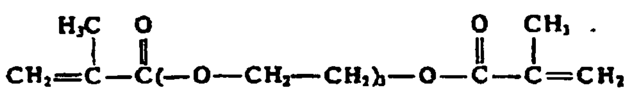

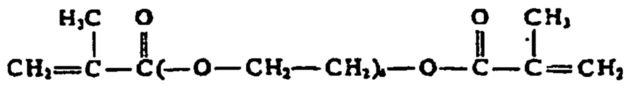

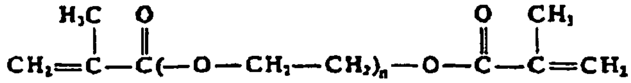

n = 9

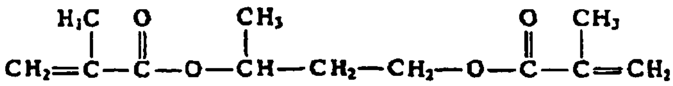

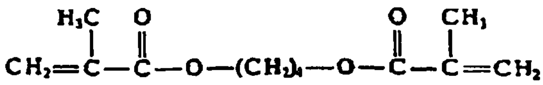

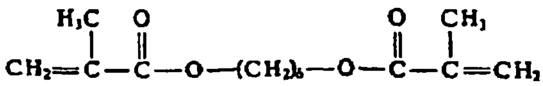

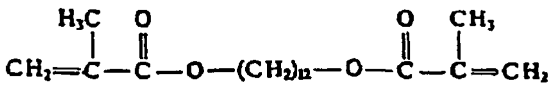

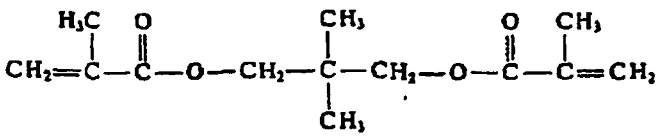

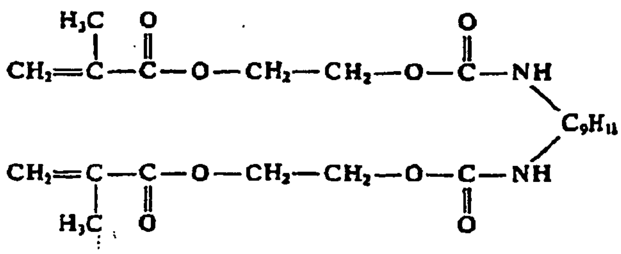

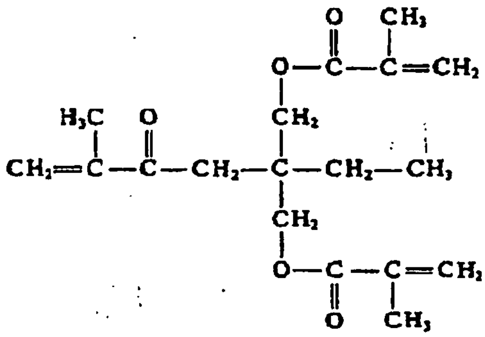

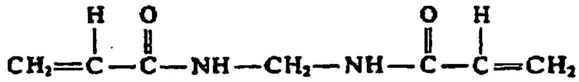

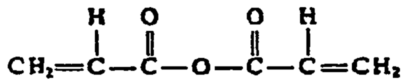

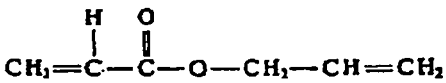

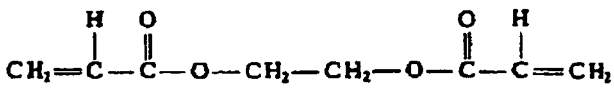

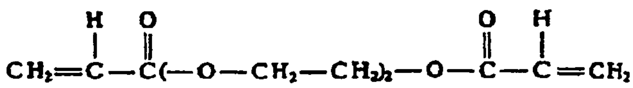

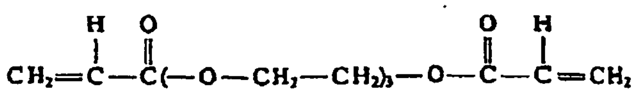

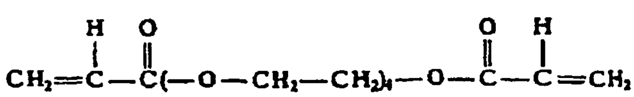

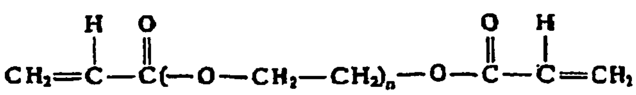

n - 9

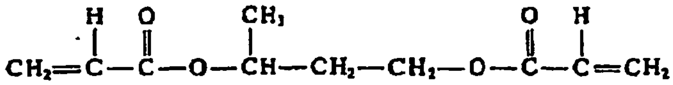

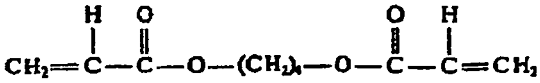

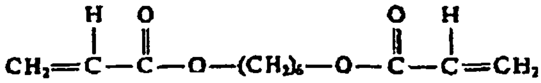

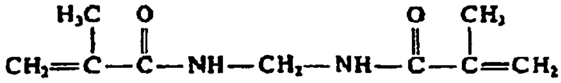

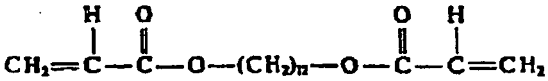

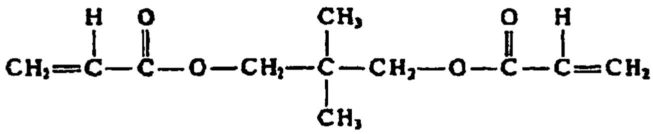

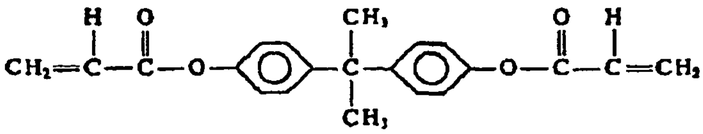

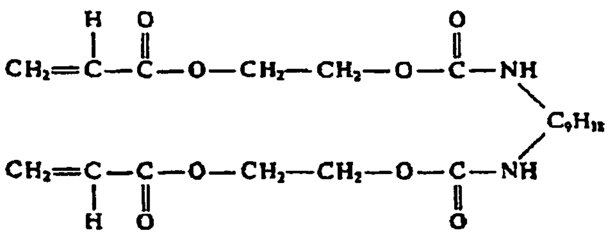

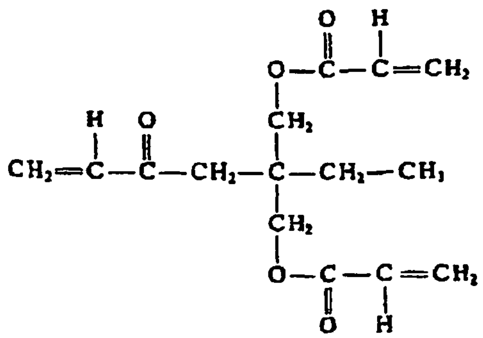

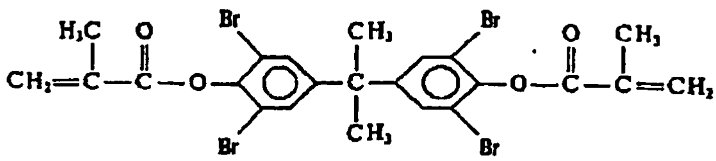

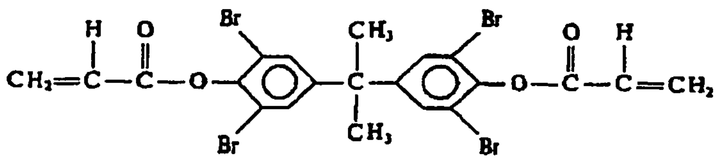

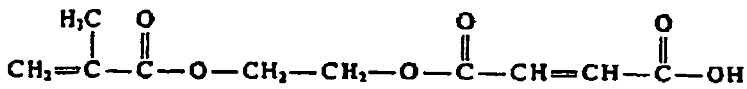

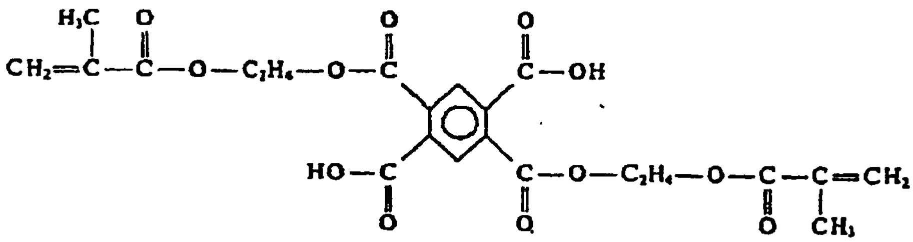

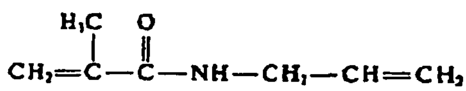

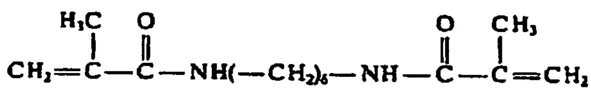

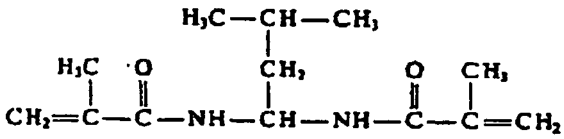

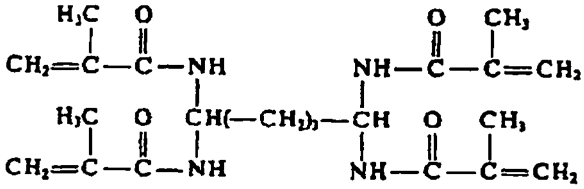

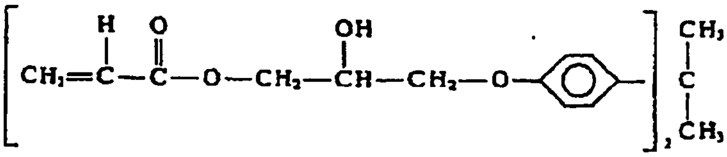

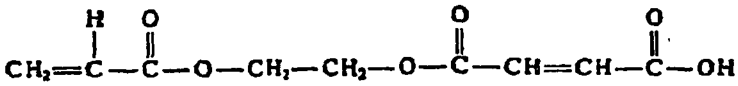

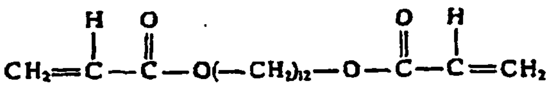

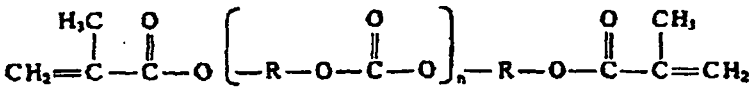

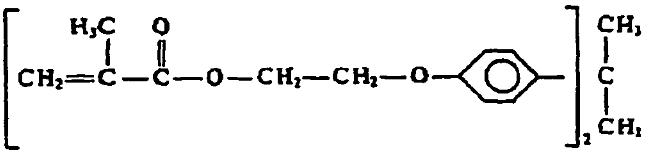

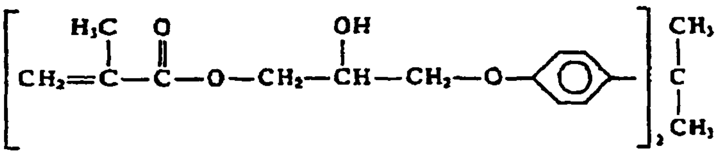

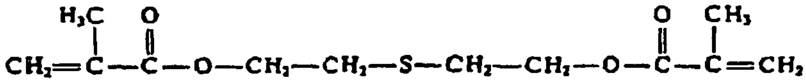

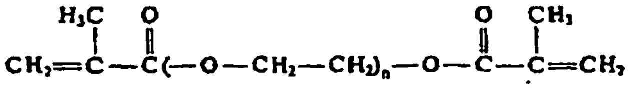

n ~ 14

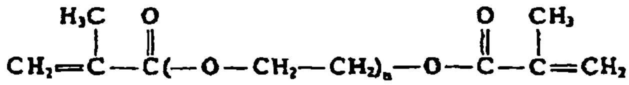

n ~ 23

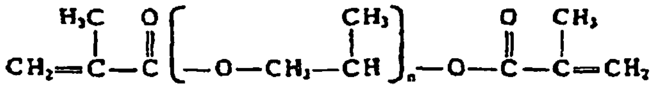

n ~ 7

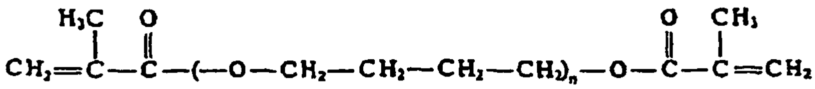

n ~ 9

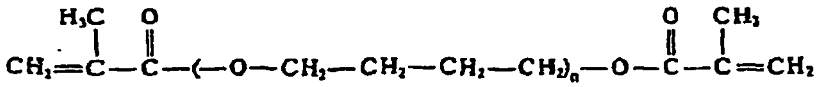

n ~ 40

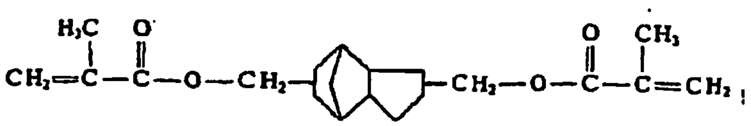

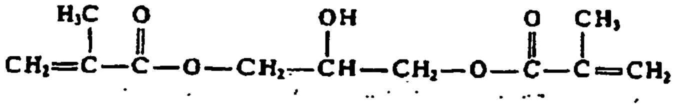

Wird in Fällen, in denen in Formel (II) n größer 1 ist, die Phosphorverbindung nicht in äquivalenter Menge zugegeben, sondern in einem Unterschuß, beispielsweise mit 1,5 Mol pro Mol einer Verbindung der Formel (II) mit n gleich 2, so erhält man Mischungen aus Verbindungen der Formel (I). Ein Bestandteil dieser Mischung wird dann in der Regel eine Verbindung der Formel (I) sein, in der n gleich 2 ist, und ein weiterer eine Verbindung der Formel (I), in der n gleich 1 ist und worin {B} eine weitere Gruppe [Y-$(CHR^5)_m$ C(O)X-] enthält, die beispielsweise mit einer Alkoholfunktion von {B} verestert sein kann.

Die am Phosphor gebundenen Alkoxygruppen lassen sich anschließend bei Bedarf durch dem Fachmann

bekannte Maßnahmen in einfacher Weise (z.B. durch Hydrolyse) in Hydroxygruppen umwandeln.

Die efindungsgemäßen Verbindungen können entweder als solche über ihre organisch polymerisierbaren Gruppen polymerisiert oder über diese Gruppen in andere Polymere eingearbeitet (polymerisiert, polyaddiert) werden. So können sie beispielsweise als Bestandteile rein organischer Copolymere dienen oder, in besonders vorteilhafter Weise, mit für ORMOCERe® vorgesehenen Materialien (hydrolytisch kondensierbaren Massen oder bereits hydrolytisch kondensierten Solen/Gelen) zusammen organisch polymerisiert werden (ORMOCERe® sind anorganisch-organische Polymermaterialien, die durch Hydrolyse von hydrolysierbaren Silanen und ggf. hydrolysierbaren Verbindungen anderer Metalle sowie ggf. eine organische Vernetzung organischer Bestandteile der genannten Silane und/oder weiterer Komponenten erhältlich sind). Die auf diese Weise erhaltenen, rein organischen oder anorganisch-organischen Polymere (Homopolymere oder hybride Werkstoffe) können beispielsweise in Form von Bulkmaterialien, Kompositen, Zementen, Klebstoffen, Vergußmassen, Beschichtungsmaterialien, Haftvermittlern oder als Bindemittel für keramisch Partikel (in keramischen Formgebungsverfahren) eingesetzt werden. Weiterhin eignen sie sich beispielsweise zur Herstellung bzw. Primung von Füllstoffen und Fasern, für Schleifscheiben oder für die Verwendung in Reaktionsextrudern oder dergleichen. Dabei kann bzw. können ihre organisch polymerisierbare(n) Gruppe(n) photochemisch, thermisch oder strahlungsinduziert vernetzt bzw. in das organische Netzwerk der Cokomponenten eingebaut werden. Ein Beispiel für mögliche Einbaureaktionen ist die Thiol-en-Addition, bei der die erfindungsgemäßen Verbindungen an Thiole addiert werden können. Alternativ sind Selbsthärtungprozesse möglich, beispielsweise kovalent-nukleophil (z.B. durch Aminohärtung) oder über Redoxreaktionen, die ggf. mit photoinduzierter bzw. thermischer Härtung kombinierbar sind.

Die erfindungsgemäßen Verbindungen besitzen vorteilhafte Eigenschaften bzw. Eigenschaftskombinationen, und diese Eigenschaften bzw. Eigenschaftskombinationen sollten auch den daraus oder damit herstellbaren Produkten (Homopolymeren oder hybriden Werkstoffen) innewohnen. Diese Eigenschaften sind sehr stark von der Art der Gruppen $R^1$ und $R^2$ abhängig. Die Natur dieser Gruppen bestimmt wesentlich die Acidität, die Leitfähigkeit, die Polarität, die Reaktivität und das Komplexierungsvermögen. Acidität, Leitfähigkeit, Polarität und Reaktivität, insbesondere auch das Ätzvermögen, und auch das Komplexierungsvermögen nehmen in der Reihe $R^1/R^2$ gleich OH/OH, OH/OR, OH/R, OR/OR, R/OR, R/R (mit R hier in der Bedeutung Alkyl, Alkenyl, Aryl, Alkylaryl oder Arylalkyl) ab. Verbindungen mit $R^1/R^2$ gleich R/OR bzw. R/R sind daher dann einsetzbar, wenn weniger agressive Verbindungen benötigt werden und schonendere Oberflächen erzeugt werden sollen. Beispielhaft sei angeführt: Die Tatsache, dass die phosphorhaltige Gruppe ein Ladungsträger sein kann (nämlich für $R^1$ und/oder $R^2$ = Hydroxy bzw. in Hydroxy überführbares Alkoxy, Aryloxy u.dgl.), ermöglicht die Herstellung wässriger Emulsionen oder Dispersionen aus bzw. mit den erfindungsgemäßen Verbindungen bzw. (Pre-)Polymeren davon, die vorteilhafte Eigenschaften bezüglich ihrer Leitfähigkeit haben, sich z.B. für die Elektrotauchlackierung eignen und antistatisch sind bzw. sein können. Die phosphorhaltige Gruppe kann katalytisch wirksam sein, weshalb sie z.B. als saurer einpolymerisierbarer Katalysator z.B. für die hydrolytische Kondensation im Sol-Gel-Verfahren zur Herstellung von (Hetero)Polykondensaten z.B. mit Si-O-Si-Brücken dienen kann. Die Anwesenheit bzw. Beigabe der erfindungsgemäßen Verbindungen läßt dabei, da sie über ihren organisch polymerisierbaren Anteil mit anderen organisch polymerisierbaren Gruppen im Polykondensat in das Netzwerk des Endproduktes eingebaut werden können, einen späteren Abtrennungsschritt des ansonsten zu verwendenden Katalysators entfallen. Aufgrund der Polarität der phosphorhaltigen Gruppe sind die erfindungsgemäßen Verbindungen weiterhin gut in polaren Medien löslich. Die phosphorhaltige Gruppe sorgt außerdem für gute Haftungseigenschaften auf anorganischen, organischen und hybriden (anorganischorganischen) Oberflächen, und zwar ebenfalls vor allem dann, wenn $R^1$ und/oder $R^2$ Hydroxy bedeuten. Ihre Fähigkeit zur Komplexierung/Bindung von Titan-, Zirkonium-, Zinn-, Calcium- und anderen Metall- und Übergangsmetallionen läßt eine Modifikation bzw. Einstellung von Eigenschaften wie der Röntgenopazität, der Brechzahl und der Kontakttoxizität der aus den erfindungsgemäßen Verbindungen hergestellten Materialien erwarten. Außerdem wirken die phosphorhaltigen Gruppen antimikrobiell, und sie können - wiederum vor allem mit Resten $R^1$ und/oder $R^2$ gleich Hydroxy - als reaktive Gruppen bei Zementbildungsreaktionen oder zum Aufwachsen von anorganischen Werkstoffen, z.B. Hydroxylapatit, eingesetzt werden, was sie für den Medizin-, insbesondere den Dentalbereich geeignet erscheinen läßt. Chemisch lassen sich die phoshorhaltigen Gruppen weiter funktionalisieren, und physikalisch bieten die mit den erfindungsgemäßen Verbindungen hergestellten Produkte eine hohe Temperaturstabilität, guten Korrosionsschutz und Flammfestigkeit (matrixinternen Flammschutz), wobei die flammhemmende Wirkung mit der Anzahl der Phosphoratome im Werkstoff zunimmt.

Für den Medizinsektor, insbesondere den Dentalbereich besonders geeignet sind Verbindungen der vorliegenden Erfindung gemäß Formel (I), in denen mindestens eine, bevorzugt beide der Gruppen $R^1$, $R^2$ OR bzw. Hydroxy bedeutet. Der Grund liegt in den oben erwähnten Eigenschaften dieser Verbindungen. Besonders bieten sich Verwendungen der daraus oder damit hergestellten Massen als Haftvermittler, als Matrixbestandteil für Zemente, zum Aufwachsen von natürlichem Zahn- oder Knochengewebe, als Komposite, Kompomere, Vergußmassen oder Klebstoffe an. Außerdem sind für die genannten Zwecke von Massen für den Einsatz im Dentalbereich besonders geeignet solche Verbindungen, die statt dessen oder zusätzlich zu den vorgenannten Merkmalen mehrere Doppelbindungen, vor allem mehrere in ein Michaelsystem eingebundene Doppelbindungen enthalten, da diese eine engmaschigere organische Vernetzung ermöglichen. Hierfür kann man auch in geeigneter Weise Mischungen von erfindungsgemäßen Verbindungen einsetzen, die z.B. wie voranstehend erläutert dadurch erhältlich sind, dass eine Verbindung der Formel (II) mit n größer 1 mit einem Unterschuß an Phosphorverbindung, bezogen auf die reaktiven Gruppen Y, umgesetzt wird. Das erhaltene Gemisch enthält dann eine Verbindung mit einer zusätzlichen Gruppe [Y-$(CHR^5)_m$ C(O)X-].

Nachstehend soll die Erfindung anhand von Beispielen näher erläutert werden.

Reaktionsschema:

(Umsetzung eines Oligo(meth-)acrylates mit einem Phosphit und anschließende Hydrolyse)

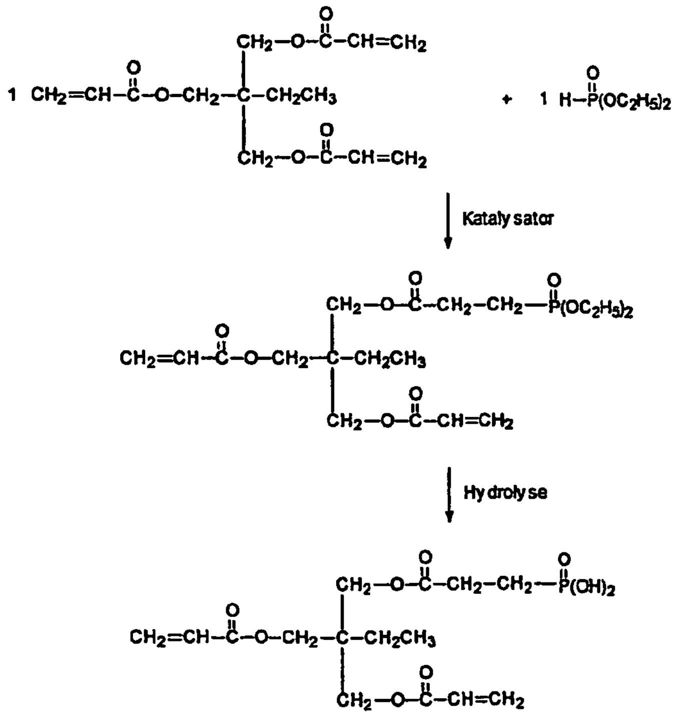


Beispiel 1

Umsetzung von Trimethylolpropantriacrylat (TMPTA) mit Diethylphosphit.

Zur Vorlage von 4,61 g (15,5 mmol) TMPTA werden unter trockener Schutzgasatmosphäre, Kühlung (exotherme Reaktion!) und Rühren zunächst 2,13 g Diethylphosphit (15,5 mmol) und anschließen eine 25%-ige Natriummethanolat-Lösung als Katalysator zugesetzt. Es findet eine schnelle Reaktion statt, die innerhalb weniger Minuten abgeschlossen ist. Der Reaktionsverlauf und somit der vollständige Umsatz dieser exothermen PH-Addition kann mittels IR-Spektroskopie anhand folgender Änderungen nachgewiesen werden:

- Verschwinden der $\nu_{PH}$-Bande bei 2426 $cm^{-1}$
- Abnahme der $\nu_{CH}$-Bande (Olefin) bei ca. 3040 $cm^{-1}$,
- Abnahme der $\nu_{C=C}$-Bande bei 1635/1619 $cm^{-1}$
- Entstehen der $\nu_{C=O}$-Ester-Bande bei 1739 $cm^{-1}$ und damit Überlagerung der $\nu_{C=O}$-Acrylat-Bande bei 1728 $cm^{-1}$, die abnimmt.

Das resultierende Phosphonat-modifizierte Acrylat (Isomerengemisch) kann durch übliche Aufarbeitung isoliert oder bevorzugt direkt weiter umgesetzt werden.

Beispiel 2

Umsetzung von Trimethylolpropantrimethacrylat (TMPTMA) mit Diethylphosphit.

Zur Vorlage von 25,9 g (76,5 mmol) TMPTMA werden unter trockener Schutzgasatmosphäre, Kühlung (exotherme Reaktion!) und Rühren zunächst 10,8 g Diethylphosphit (76,5 mmol) und anschließend eine 25%-ige Natriummethanolat-Lösung als Katalysator zugesetzt. Es findet eine schnelle Reaktion statt, die innerhalb weniger Minuten

abgeschlossen ist. Der Reaktionsverlauf und somit der vollständige Umsatz dieser exothermen PH-Addition kann mittels IR-Spektroskopie anhand folgender Änderungen nachgewiesen werden:

- Verschwinden der $\nu_{PH}$-Bande bei 2426 $cm^{-1}$
- Abnahme der $\nu_{CH}$-Bande (Olefin) bei ca. 3040 $cm^{-1}$
- Abnahme der $\nu_{C=C}$-Bande bei 1638 $cm^{-1}$.
- Entstehen der $\nu_{C=O}$-Ester-Bande bei 1738 $cm^{-1}$ und damit Überlagerung der $\nu_{C=O}$-Methacrylat-Bande bei 1721 $cm^{-1}$ die abnimmt.

Das resultierende Phosphonat-modifizierte Methacrylat (Isomerengemisch) kann durch übliche Aufarbeitung isoliert oder bevorzugt direkt weiter umgesetzt werden.

Beispiel 3

Umsetzung von Glycerinacrylatdimethacrylat mit Diethylphosphit

Zu einem Gemisch von 32 g (0.1133 mol) Glycerinacrylatdimethacrylat und 15,85 g (0,1133 mol) Diethylphosphit wird unter trockener Schutzgasatmosphäre und Rühren ein basischer Katalysator langsam zugetropft. Es findet eine schnelle Reaktion mit vollständigem Umsatz des Diethylphosphits, sichtbar am Verschwinden der $\nu_{PH}$-Bande bei 2426 $cm^{-1}$ statt. Das resultierende, flüssig, doppelbindungshaltige Phosphonat kann durch übliche Aufarbeitung isoliert werden. Die erfolgreiche durchgeführte Synthese der Phosphonatverbindung kann mit Hilfe von $^1H$-, $^{13}C$- und FT-IR-Spektroskopie nachgewiesen werden. Folgende NMR-Daten für das Acrylat-addierte Phosphonat werden erhalten:
$^1H$-NMR (400 MHz, $CDCl_3$, δ [ppm]): 1,32 (t, 6H, $P(OCH_2CH_3)_2$), 1,94 (s, 6H, 2x=$CCH_3$-), 2,06 (m, 2H, $PCH_2CH_2$-), 2,62 (m. 2H, $PCH_2CH_2$-), 4,0-4,6 (m, 8H, $P(OCH_2CH_3)_2$, -$OCH_2CH(O-)CH_2O$-), 5,38 (1H, -$OCH_2CH(O-)CH_2O$-), 5,61 und 6,11 (s, 4H, 2x$CH_2$=)
$^{13}C$-NMR (100 MHz, $CDCl_3$, δ [ppm]): 16,70 (P $OCH_2CH_3$), 18,25 (=$CHCH_3$-), 20,23 und 21,67 ($PCH_2CH_2$-), 27,54 ($PCH_2CH_2$-), 61,85 ($POCH_2CH_3$), 62,42 (-$OCH_2CH(O-)CH_2O$-),
126,43 und 135,66 (C=$CH_2$), 166,72 ($CH_2$=$C(CH_3)COO$-), 174,76 ($PCH_2CH_2COO$-)

IR (Film):

- $\nu_{PH}$-Bande bei 2426 $cm^{-1}$ verschwindet bei der Umsetzung
- $\nu_{CH}$-Bande (Olefin) bei ca. 3040 $cm^{-1}$ nimmt ab
- $\nu_{C=C}$-Bande bei 1638 $cm^{-1}$ nimmt ab
- Entstehung der $\nu_{C=O}$-Ester-Bande bei 1746 $cm^{-1}$ und damit Überlagerung der $\nu_{C=O}$-Methacrylat-Bande bei 1724 $cm^{-1}$ die abnimmt.

Beispiel 4

Hydrolyse der Phosphonatverbindung aus Beispiel 3

Zu einer Lösung von 15 g (0,0356 mol) Phosphonatverbindung (Beispiel 4) in 20 ml wasserfreiem Methylenchlorid werden bei Raumtemperatur unter Schutzgasatmosphäre und Rühren 11,76 ml (0,0891 mol) Trimethylsilyibromid langsam zugetropft. Der Ansatz wird für 24 Stunden bei Raumtemperatur gerührt. Anschließend wird das Reaktionsgemisch eingeengt, mit 15 ml absolutem Methanol unter Rühren versetzt und für 4 Stunden bei Raumtemperatur gerührt. Das Lösungsmittel wird danach im Vakuum abgezogen und das flüssige Produkt erhalten. Die Phosphonsäure und damit die Vollständigkeit der Hydrolyse kann mit Hilfe von $^1H$-,$^{13}C$- und FT-IR-Spektroskopie nachgewiesen werden.
$^1H$-NMR (400 MHz, $CDCl_3$, δ [ppm]): 1,93 (s, 6H, 2x-$CCH_3$-), 2,09 (m, 2H, $PCH_2CH_2$-), 2,64 (m, 2H, $PCH_2CH_2$-), 4,0-4,60 (m, 4H, -$OCH_2CH(O-)CH_2O$-), 5,38 (1H, -$OCH_2CH(O-)CH_2O$-) 5,61 und 6,11 (s, 4H, 2x$CH_2$=), 9,97 (s, POH)
$^{13}C$-NMR (100 MHz, $CDCl_3$, δ [ppm]): 18,23 (=$CHCH_3$-), 20,81 und 22,27 ($PCH_2CH_2$-), 27,44 ($PCH_2CH_2$-), 62.44 (-$OCH_2CH(O-)CH_2O$-), 126,58 und 135,62 (C=$CH_2$), 166,86 ($CH_2$=$C(CH_3)COO$-), 171,55 ($PCH_2CH_2COO$-)
IR (Film): $\nu_{C=C}$-Banden bei 1638 $cm^{-1}$ $\nu_{C=O}$-Ester-Bande bei 1746 $cm^{-1}$; $\nu_{C=O}$-Methacrylat-Bande bei 1724 $cm^{-1}$

Beispiel 5

Polymerisation der Phosphonsäure aus Beispiel 4 zur Herstellung von Formkörpern

Die Phosphonsäure aus Beispiel mit 1% Lucirin TPO wird in eine Härtungsform (z. B. 2 x 2 x 25 $mm^3$) gegeben. Die Methacrylatgruppen werden im Rahmen einer photoinduzierten radikalischen Polymerisation umgesetzt, wobei das Harz aushärtet.

Beispiel 6

Flammtest mit Formkörpern aus Beispiel 5

Nach Beflammung (ca. 10 sec lang) der Formkörper aus Beispiel 6 mit einer ca. 900°C Flamme wird nach Entfernung der Flamme eine augenblickliche Selbstverlöschung beobachtet. Ein entsprechender Formkörper ohne die Phosphonsäuregruppen, d. h. auf rein organischer Basis verbrennt hingegen vollständig mit großer Flamme.

Beispiel 7

Polymerisation der Phosphonsäure aus Beispiel 6 zur Herstellung einer haftvermittelnden Schichten

Die Phosphonsäure aus Beispiel mit 1% Lucirin TPO wird z. B. auf Dentalgewebe aufgetragen. Die Methacrylatgruppen werden im Rahmen einer phatoinduzierten radikalischen Polymerisation umgesetzt, wobei das Harz (Haftvermittler für ein dentales Komposit) aushärtet.

Beispiel 8

Synthese des Mono-Phosphinsäureethylester auf Basis von TMPTA

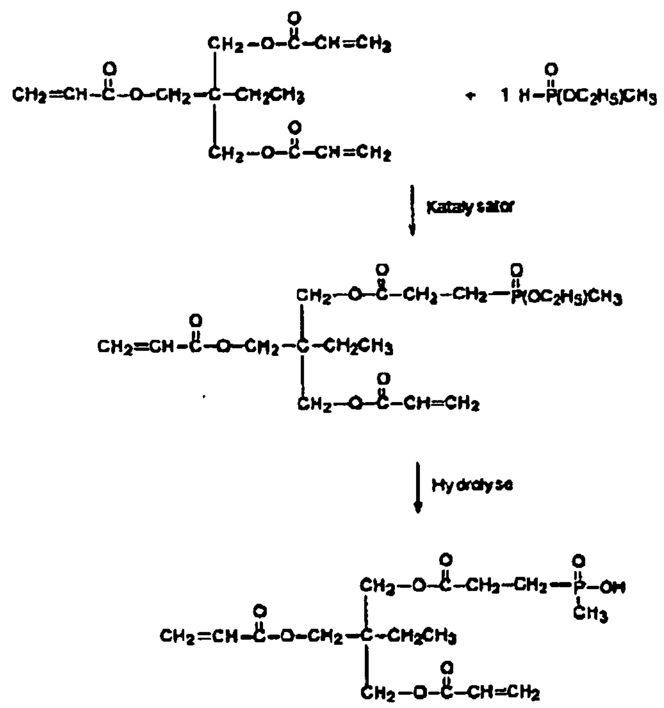


Zur Vorlage von 5,95 g (20,0 mmol) Trimethylolpropantriscrylat (TMPTA) wird unter trockener Schutzgasatmosphäre und Rühren zunächst 2,16 g Methylphosphinsäureethylester (20.0 mmol) und anschließen ein basischer Katalysator zugesetzt. Es findet eine schnelle Reaktion, mit vollständigem Umsatz des Phosphinsäureesters, sichtbar am Verschwinden der $\nu_{PH}$-Bande, statt. Der Reaktionsverlauf und somit der vollständige Umsatz dieser PH-Addition kann wie üblich (siehe Beispiel 1) mittels IR-Spektroskopie nachgewiesen werden.

Das resultierende Phosphonat-modifizierte Acrylat (Isomerengemisch) kann durch übliche Aufarbeitung isoliert werden und nach üblichen Verfahren (s. z. B. Beispiel 5) hydrolysiert werden.

## Patentansprüche

1. Verbindungen der Formel (I)

$$[(R^1)(R^2)P(O)-(CHR^3)-(CHR^4)-(CHR^5)_m-C(O)X-]_n\{B\} \qquad I$$

worin die Reste und Indices die folgende Bedeutung haben:

X ist O, NH, $NR^6$ oder S,
$R^1$, $R^2$ sind unabhängig voneinander Wasserstoff oder ggf. substituiertes Alkyl, Alkenyl, Aryl, Alkylaryl oder Arylalkyl oder ggf. substituiertes Alkoxy, Aryloxy, Alkylaryloxy, Arylalkyloxy oder Hydroxy
$R^3$, $R^4$, $R^6$ und $R^6$ sind unabhängig voneinander Wasserstoff oder ggf. substituiertes Alkyl, Alkenyl, Aryl, Alkylaryl oder Arylalkyl
{B} ist ein geradkettiger oder verzweigter Rest, der mindestens zwei Vinyl-, Allyl-, Norbomen-, Glycidyl-, Acrylat-, Metacrylat-, Thioacrylat- oder Thiomethacrylat-Gruppen oder von (Meth)Acrylsäureamiden abgeleitete Gruppen aufweist und der kein Siliciumatom enthält,
m ist eine ganze Zahl von 0 bis 20,
n ist eine ganze Zahl von 1 bis 20
ausgenommen Verbindungen, in denen {B} eine oder mehrere Isolierte oder oligomerisierte Isoprenguppe(n) aufweist.

2. Verbindungen der Formel (I) nach Anspruch 1, **dadurch gekennzeichnet, dass** $R^1$ und $R^2$ beide Hydroxy sind oder dass $R^1$ und $R^2$ beide ggf. substituiertes Alkoxy, Alkenyloxy, Aryloxy, Alkylaryloxy oder Arylalkyloxy sind oder dass $R^1$ ggf.
substituiertes Alkoxy, Alkenyloxy, Aryloxy, Alkylaryloxy oder Arylalkyloxy ist und $R^2$ ggf. substituiertes Alkyl, Alkenyl, Aryl, Alkylaryl oder Arylalkyl ist oder dass $R^1$ OH ist und $R^2$ ggf. substituiertes Alkyl, Alkenyl, Aryl, Alkylaryl oder Arylalkyl ist oder
dass $R^1$ und $R^2$ beide Wasserstoff oder ggf. substituiertes Alkyl, Alkenyl, Aryl,
Alkylaryl oder Arylalkyl sind.

3. Verbindungen der Formel (I) nach einem der voranstehenden Ansprüche, **dadurch gekennzeichnet, dass** X NH oder $NR^6$ oder Sauerstoff ist und besonders bevorzugt Sauerstoff ist.

4. Verbindungen der Formel (I) nach einem der voranstehenden Ansprüche, **dadurch gekennzeichnet, dass** m 0, 1, 2, 3 oder 4 ist und/oder dass n 1, 2, 3 oder 4 ist.

5. Verbindungen der Formel (I) nach Anspruch 4, **dadurch gekennzeichnet, dass** m 0 ist und/oder dass n 1 ist.

6. Verbindungen der Formel (I) nach einem der voranstehenden Ansprüche, **dadurch gekennzeichnet, dass** {B} mindestens 4 Kohlenstoffatome aufweist.

7. Verbindungen der Formel (I) nach einem der voranstehenden Anspruch, **dadurch gekennzeichnet, dass** X Sauerstoff bedeutet.

8. Verbindungen der Formel (I) nach Anspruch 7, **dadurch gekennzeichnet, dass** der Rest {B} mindestens zwei Michaelsysteme, insbesondere Acrylat-, Methacrylatgruppen, und/oder Glycidylgruppe(n) enthält, wobei ggf. {B} ein von einem Oligoalkohol abgeleitetes Kohlenstoffgerüst enthält, wobei eine oder mehrere der Hydroxyfunktionen des Oligoalkohols mit einer bzw. mehreren Acrylat- und/oder Methacrylatgruppen verestert ist/sind.

9. Verbindungen der Formel (I) nach Anspruch 8, **dadurch gekennzeichnet, dass** der Oligoalkohol ein Kohlostoffgerüst mit drei bis 15 Kohlenstoffatomen aufweist.

10. Verbindungen der Formel (I) nach einem der Ansprüche 8 oder 9, **dadurch gekennzeichnet, dass** X Sauerstoff ist und als weitere Hydroxyfunktion des Oligoalkohols aufgefasst werden kann.

11. Verbindungen der Formel (I) nach Anspruch 10, **dadurch gekennzeichnet, dass** {B} über das genannte von einem Oligoalkohol abgeleitete Kohlenstoffgerüst und die damit veresterten Acrylat- und/oder Methacrylatgruppen hinaus keine weiteren Gruppen aufweist.

12. Verfahren zum Herstellen von Verbindungen der Formel (I) wie in einem der voranstehenden Ansprüche definiert, **dadurch gekennzeichnet, dass** man Verbindungen der Formel (II)

$[Y\text{-}(CHR^5)_m\ C(C)X\text{-}]_n\{B\}$ II

mit Verbindungen der Formel (III)

$(R^1)(R^2)P(O)H$ III

umsetzt, wobei die Reste und Indices wie in Anspruch 1 für Formel (I) definiert sind und Y eine organische Gruppe ist, aus der mit dem an das Phosphoratom gebundene Wasserstoff der Verbindung (III) eine $(CHR^3)\text{-}(CHR^4)$-Gruppe entsteht.

**13.** Verfahren nach Anspruch 12, **dadurch gekennzeichnet, dass** die Reste $R^1$ und $R^2$ $C_1$-$C_6$-Alkoxy, vorzugsweise Methoxy oder Ethoxy bedeuten.

**14.** Verfahren nach Anspruch 13 zur Herstellung von Verbindungen mit der Formel (I), worin die Reste $R^1$ und $R^2$ Hydroxy bedeuten, **dadurch gekennzeichnet, dass** das Produkt der Umsetzung der Verbindung mit der Formel (II) mit der Verbindung der Formel (III) einer Hydrolyse unterworfen wird.

**15.** Verfahren nach einem der Ansprüche 12 bis 14, **dadurch gekennzeichnet, dass** n in der Verbindung mit der Formel (II) größer 1, vorzugsweise 2 oder 3 ist, und dass man 1 Mol dieser Verbindung mit weniger als n Mol der Verbindung mit der Formel (III) umsetzt, wobei n dieselbe Bedeutung wie in Formel (II) besitzt, um ein Gemisch mit einer Verbindung der Formel (I), in der n größer 1, vorzugsweise 2 oder 3 bedeutet, und einer Verbindung der Formel (II), in der n 1 bedeutet und worin die Gruppe {B} mindestens einen Rest $[Y\text{-}(CHR^5)_m\ C(O)X\text{-}]$ enthält, zu erhalten.

**16.** Homopolymere einer Verbindung der Formel (I)

$[(R^1)(R^2)P(O)\text{-}(CHR^3)\text{-}(CHR^4)\text{-}(CHR^5)m\text{-}C(O)X\text{-}]_n\{B\}$ I

worin die Reste und Indices die folgende Bedeutung haben:

X ist O, NH, $NR^6$ oder S,
$R^1$, $R^2$ sind unabhängig voneinander Wasserstoff oder ggf. substituiertes Alkyl, Alkenyl, Aryl, Alkylaryl oder Arylalkyl oder ggf. substituiertes Alkoxy, Aryloxy, Alkylaryloxy, Arylalkyloxy oder Hydroxy
$R^3$, $R^4$, $R^5$ und $R^6$ sind unabhängig voneinander Wasserstoff oder ggf.
substituiertes Alkyl, Alkenyl, Aryl, Alkylaryl oder Arylalkyl
{B} ist ein geradkettiger oder verzweigter Rest mit mehr als einer organisch polymerisierbaren Gruppe und mindestens 2 Kohlenstoffatomen, der kein Siliciumatom enthält,
m ist eine ganze Zahl von 0 bis 20,
n ist eine ganze Zahl von 1 bis 20.

**17.** Homopolymere nach Anspruch 16 worin die in Formel (I) angegebenen Reste und Indices die in einem der Ansprüche 2 bis 11 angegebene Bedeutung besitzen.

**18.** Mischpolymere von verschiedenen Verbindungen der Formel (I)

$[(R^1)(R^2)P(O)\text{-}(CHR^3)\text{-}(CHR^4)\text{-}(CHR^6)_m\text{-}C(O)X\text{-}]_n\{B\}$ I

worin die Reste und Indices die folgende Bedeutung haben:

X ist O, NH, $NR^6$ oder S,
$R^1$, $R^2$ sind unabhängig voneinander Wasserstoff oder ggf. substituiertes Alkyl, Alkenyl, Aryl, Alkylaryl oder Arylalkyl oder ggf. substituiertes Alkoxy, Aryloxy, Alkylaryloxy, Arylalkyloxy oder Hydroxy
$R^3$, $R^4$, $R^5$ und $R^6$ sind unabhängig voneinander Wasserstoff oder ggf.
substituiertes Alkyl, Alkenyl, Aryl, Alkylaryl oder Arylalkyl
{B} ist ein geradkettiger oder verzweigter Rest mit mehr als einer organisch polymerisierbaren Gruppe und mindestens 2 Kohlenstoffatomen, der kein Siliciumatom enthält,
m ist eine ganze Zahl von 0 bis 20,
n ist eine ganze Zahl von 1 bis 20.

**19.** Mischpolymere nach Anspruch 18, worin die in Formel (I) angegebenen Reste und Indices die in einem der Ansprüche 2 bis 11 angegebene Bedeutung besitzen.

**20.** Copolymerisate, gebildet unter Verwendung von Monomereinheiten der Formel (I) oder von Blockpolymer-Einheiten, die aus Monomeren der Formel (I) aufgebaut sind,

$$[(R^1)(R^2)P(O)\text{-}(CHR^3)\text{-}(CHR^4)\text{-}(CHR^5)_m\text{-}C(O)X\text{-}]_n\{B\} \qquad I$$

worin die Reste und Indices die folgende Bedeutung haben:

X ist O, NH, $NR^6$ oder S,
$R^1$, $R^2$ sind unabhängig voneinander Wasserstoff oder ggf. substituiertes Alkyl, Alkenyl, Aryl, Alkylaryl oder Aralkyl oder ggf. substituiertes Alkoxy, Aryloxy, Alkylaryloxy, Arylalkyloxy oder Hydroxy
$R^3$, $R^4$, $R^5$ und $R^6$ sind unabhängig voneinander Wasserstoff oder ggf.
substituiertes Alkyl, Alkenyl, Aryl, Alkylaryl oder Arylalkyl
{B} ist ein geradkettiger oder verzweigter Rest mit mehr als einer organisch polymerisierbaren Gruppe und mindestens 2 Kohlenstoffatomen, der kein Siliciumatom enthält,
m ist eine ganze Zahl von 0 bis 20,
n ist eine ganze Zahl von 1 bis 20.

**21.** Copolymerisate nach Anspruch 20, worin die in Formel (I) angegebenen Reste und Indices die in einem der Ansprüche 2 bis 11 angegebene Bedeutung besitzen.

## Claims

**1.** Compounds of formula (I)

$$[(R^1)(R^2)P(O)\text{-}(CHR^3)\text{-}(CHR^4)\text{-}(CHR^5)_m\text{-}C(O)X\text{-}]_n\{B\} \qquad I$$

in which the residues and indices have the following meanings:

X is O, NH, $NR^6$ or S;
$R^1$, $R^2$ are, independently of one another, hydrogen or optionally substituted alkyl, alkenyl, aryl, alkylaryl, or arylalkyl or optionally substituted alkoxy, aryloxy, alkylaryloxy, arylalkyloxy, or hydroxy;
$R^3$, $R^4$, $R^5$, and $R^6$ are, independently of one another, hydrogen or optionally substituted alkyl, alkenyl, aryl, alkylaryl, or arylalkyl;
{B} is a straight-chain or branched residue which comprises at least two vinyl, allyl, norbornene, glycidyl, acrylate, methacrylate, thioacrylate, or thiomethacrylate groups or groups derived from (meth)acrylamides and which comprises no silicon atom;
m is an integer from 0 to 20;
n is an integer from 1 to 20,
apart from compounds in which {B} comprises one or more isolated or oligomerized isoprene group(s).

**2.** Compounds of formula (I) according to claim 1, **characterized in that** $R^1$ and $R^2$ are both hydroxy or that $R^1$ and $R^2$ are both optionally substituted alkoxy, alkenyloxy, aryloxy, alkylaryloxy, or arylalkyloxy or that $R^1$ is optionally substituted alkoxy, alkenyloxy, aryloxy, alkylaryloxy, or arylalkyloxy and $R^2$ is optionally substituted alkyl, alkenyl, aryl, alkylaryl, or arylalkyl or that $R^1$ is OH and $R^2$ is optionally substituted alkyl, alkenyl, aryl, alkylaryl, or arylalkyl or that $R^1$ and $R^2$ are both hydrogen or optionally substituted alkyl, alkenyl, aryl, alkylaryl or arylalkyl.

**3.** Compounds of formula (I) according to one of the preceding claims, **characterized in that** X is NH or $NR^6$ or oxygen and particularly preferably is oxygen.

**4.** Compounds of formula (I) according to one of the preceding claims, **characterized in that** m is 0, 1, 2, 3, or 4 and/or that n is 1, 2, 3, or 4.

**5.** Compounds of formula (I) according to claim 4, **characterized in that** m is 0 and/or that n is 1.

**6.** Compounds of formula (I) according to one of the preceding claims, **characterized in that** {B} comprises at least 4 carbon atoms.

**7.** Compounds of formula (I) according to one of the preceding claims, **characterized in that** X represents oxygen.

**8.** Compounds of formula (I) according to claim 7, **characterized in that** said residue {B} comprises at least two Michael systems, in particular acrylate, methacrylate groups, and/or glycidyl group(s), wherein, optionally, {B} comprises a carbon backbone derived from an oligoalcohol, one or more of the hydroxy functions of the oligoalcohol being esterified with one or more acrylate and/or methacrylate groups.

**9.** Compounds of formula (I) according to claim 8, **characterized in that** said oligoalcohol comprises a carbon backbone having three to 15 carbon atoms.

**10.** Compounds of formula (I) according to one of claims 8 or 9, **characterized in that** X is oxygen and can be considered as a further hydroxy function of said oligoalcohol.

**11.** Compounds of formula (I) according to claim 10, **characterized in that** {B} comprises no further groups besides said carbon backbone derived from an oligoalcohol and said acrylate and/or methacrylate groups esterified therewith.

**12.** Method of preparing compounds of formula (I) as defined in one of the preceding claims, **characterized in that** compounds of formula (II)

$$[Y\text{-}(CHR^5)_mC(O)X\text{-}]_n\{B\} \qquad II$$

are caused to react with compounds of formula (III)

$$(R^1)(R^2)P(O)H \qquad III$$

wherein the residues and indices are defined as in claim 1 for formula (I) and Y is an organic group from which a $(CHR^3)$-$(CHR^4)$-group is formed with the hydrogen bound to the phosphorus atom of compound (III).

**13.** Method according to claim 12, **characterized in that** said residues $R^1$ and $R^2$ represent $C_1$-$C_6$-alkoxy, preferably methoxy or ethoxy.

**14.** Method according to claim 13 of preparing compounds of formula (I) in which said residues $R^1$ and $R^2$ represent hydroxy, **characterized in that** the product of the reaction of said compound of formula (II) with said compound of formula (III) is subjected to a hydrolysis.

**15.** Method according to one of claims 12 to 14, **characterized in that** in said compound of formula (II), n is greater than 1, preferably is 2 or 3, and that 1 mol of said compound is caused to react with less than n mol of said compound of formula (III), n having the same meaning as in formula (II), in order to obtain a mixture having a compound of formula (I) in which n is greater than 1, preferably is 2 or 3, and a compound of formula (II) in which n is 1 and in which said group {B} comprises at least one residue $[Y\text{-}(CHR^5)_mC(O)X\text{-}]$.

**16.** Homopolymers of a compound of formula (I)
$[(R^1)(R^2)P(O)\text{-}(CHR^3)\text{-}(CHR^4)\text{-}(CHR^5)_m\text{-}C(O)X\text{-}]_n\{B\}$ I in which the residues and indices have the following meanings:

X is O, NH, $NR^6$ or S;
$R^1$, $R^2$ are, independently of one another, hydrogen or optionally substituted alkyl, alkenyl, aryl, alkylaryl, or arylalkyl or optionally substituted alkoxy, aryloxy, alkylaryloxy, arylalkyloxy, or hydroxy;
$R^3$, $R^4$, $R^5$, and $R^6$ are, independently of one another, hydrogen or optionally substituted alkyl, alkenyl, aryl, alkylaryl, or arylalkyl;
{B} is a straight-chain or branched residue having more than one organically polymerizable group and at least 2 carbon atoms, which comprises no silicon atom;
m is an integer from 0 to 20;
n is an integer from 1 to 20.

**17.** Homopolymers according to claim 16 wherein said residues and indices indicated in formula (I) have the meanings specified in one of claims 2 to 11.

**18.** Copolymers of different compounds of formula (I)

$$[(R^1)(R^2)P(O)\text{-}(CHR^3)\text{-}(CHR^4)\text{-}(CHR^5)_m\text{-}C(O)X\text{-}]_n\{B\} \qquad I$$

in which the residues and indices have the following meanings:

X is O, NH, $NR^6$ or S;
$R^1$, $R^2$ are, independently of one another, hydrogen or optionally substituted alkyl, alkenyl, aryl, alkylaryl, or arylalkyl or optionally substituted alkoxy, aryloxy, alkylaryloxy, arylalkyloxy, or hydroxy;
$R^3$, $R^4$, $R^5$, and $R^6$ are, independently of one another, hydrogen or optionally substituted alkyl, alkenyl, aryl, alkylaryl, or arylalkyl;
{B} is a straight-chain or branched residue having more than one organically polymerizable group and at least 2 carbon atoms, which comprises no silicon atom;
m is an integer from 0 to 20;
n is an integer from 1 to 20.

**19.** Copolymers according to claim 18 wherein said residues and indices indicated in formula (I) have the meanings specified in one of claims 2 to 11.

**20.** Copolymers formed using monomer units of formula (I) or block polymer units made up of monomers of formula (I)

$$[(R^1)(R^2)P(O)\text{-}(CHR^3)\text{-}(CHR^4)\text{-}(CHR^5)_m\text{-}C(O)X\text{-}]_n\{B\} \qquad I$$

in which the residues and indices have the following meanings:

X is O, NH, $NR^6$ or S;
$R^1$, $R^2$ are, independently of one another, hydrogen or optionally substituted alkyl, alkenyl, aryl, alkylaryl, or arylalkyl or optionally substituted alkoxy, aryloxy, alkylaryloxy, arylalkyloxy, or hydroxy;
$R^3$, $R^4$, $R^5$, and $R^6$ are, independently of one another, hydrogen or optionally substituted alkyl, alkenyl, aryl, alkylaryl, or arylalkyl;
{B} is a straight-chain or branched residue having more than one organically polymerizable group and at least 2 carbon atoms, which comprises no silicon atom;
m is an integer from 0 to 20;
n is an integer from 1 to 20.

**21.** Copolymers according to claim 20 wherein said residues and indices indicated in formula (I) have the meanings specified in one of claims 2 to 11.

## Revendications

**1.** Composés de formule (I)

$$[(R^1)(R^2)P(O)\text{-}\ (CHR^3)\text{-}(CHR^4)\text{-}(CHR^5)_m\text{-}C(O)X\text{-}]_n\{B\} \qquad I$$

dans laquelle les radicaux et indices ont la signification suivants :

X est O, NH, $NR^6$ ou S,
$R^1$, $R^2$ sont, indépendamment l'un de l'autre, l'hydrogène ou un groupe alkyle, alcényle, aryle, alkylaryle ou arylalkyle éventuellement substitué ou un groupe alcoxy, aryloxy, alkylaryloxy, arylalkyloxy ou hydroxy éventuellement substitué,
$R^3$, $R^4$, $R^5$ et $R^6$ sont, indépendamment les uns des autres, l'hydrogène ou un groupe alkyle, alcényle, aryle, alkylaryle ou arylalkyle éventuellement substitué,
{B} est un radical à chaîne droite ou ramifiée présentant au moins deux groupes vinyle, allyle, norbornène, glycidyle, acrylate, méthacrylate, thioacrylate ou thiométhacrylate ou des groupes dérivés d'amides d'acide

(méth)acrylique et ne contenant pas d'atomes de silicium,
m est un nombre entier de 0 à 20,
n est un nombre entier de 1 à 20,
à l'exception des composés dans lesquels {B} présente un ou plusieurs groupes isoprène isolés ou oligomérisés.

2. Composés de formule (I) selon la revendication 1, **caractérisés en ce que** $R^1$ et $R^2$ sont tous deux un groupe hydroxy ou **en ce que** $R^1$ et $R^2$ sont tous deux un groupe alcoxy, alcényloxy, aryloxy, alkylaryloxy ou arylalkyloxy éventuellement substitué ou **en ce que** $R^1$ est un groupe alcoxy, alcényloxy, aryloxy, alkylaryloxy ou arylalkyloxy éventuellement substitué et $R^2$ est un groupe alkyle, alcényle, aryle, alkylaryle ou arylalkyle éventuellement substitué ou bien **en ce que** $R^1$ est OH et $R^2$ est un groupe alkyle, alcényle, aryle alkylaryle ou arylalkyle éventuellement substitué ou **en ce que** $R^1$ et $R^2$ sont tous deux l'hydrogène ou un groupe alkyle, alcényle, aryle, alkylaryle ou arylalkyle éventuellement substitué.

3. Composés de formule (I) selon l'une quelconque des revendications précédentes, **caractérisés en ce que** X est NH ou $NR^6$ ou l'oxygène, l'oxygène étant particulièrement préféré.

4. Composés de formule (I) selon l'une quelconque des revendications précédentes, **caractérisés en ce que** m est 0, 1, 2, 3 ou 4 et/ou **en ce que** n est 1, 2, 3 ou 4.

5. Composés de formule (I) selon la revendication 4, **caractérisés en ce que** m est 0 et/ou **en ce que** n est 1.

6. Composés de formule (I) selon l'une quelconque des revendications précédentes, **caractérisés en ce que** {B} présente au moins 4 atomes de carbone.

7. Composés de formule (I) selon l'une quelconque des revendications précédentes, **caractérisés en ce que** X désigne l'oxygène.

8. Composés de formule (I) selon la revendication 7, **caractérisés en ce que** le radical {B} contient au moins deux systèmes de Michael, notamment des groupes acrylate-méthacrylate et/ou un ou plusieurs groupes glycidyle, le cas échéant {B} contenant un squelette carboné dérivé d'un oligoalcool, une ou plusieurs des fonctions hydroxy de l'oligoalcool étant estérifiée(s) avec un ou plusieurs groupes acrylate et/ou méthacrylate.

9. Composés de formule (I) selon la revendication 8, **caractérisés en ce que** l'oligoalcool présente un squelette carboné ayant 3 à 15 atomes de carbone.

10. Composés de formule (I) selon l'une des revendications 8 ou 9, **caractérisés en ce que** X est l'oxygène et peut être envisagé comme autre fonction hydroxy de l'oligoalcool.

11. Composés de formule (I) selon la revendication 10, **caractérisé en ce que** {B} ne présente pas d'autres groupes sur le squelette carboné susmentionné dérivé d'un oligoalcool et les groupes acrylate et/ou méthacrylate ainsi estérifiés.

12. Procédé pour préparer des composés de formule (I) comme défini dans l'une des revendications précédentes, **caractérisé en ce que** l'on fait réagir des composés de formule (II)

$$[Y\text{-}CHR^5)_mC(O)X\text{-}]_n\{B\} \qquad II$$

avec des composés de formule (III)

$$(R^1)(R^2)P(O)H \qquad III$$

les radicaux et indices étant définis comme dans la revendication 1 pour la formule (I) et Y étant un groupe organique à partir duquel on forme un groupe $(CHR^3)$-$(CHR^4)$ avec l'oxygène du composé (III) fixé à l'atome de phosphore.

13. Procédé selon la revendication 12, **caractérisé en ce que** les radicaux $R^1$ et $R^2$ désignent un groupe alcoxy en $C_1$-$C_6$, de préférence un groupe méthoxy ou éthoxy.

14. Procédé selon la revendication 13, pour produire des composés de formule (I), les radicaux $R^1$ et $R^2$ désignant un

groupe hydroxy, **caractérisé en ce qu'**on soumet à une hydrolyse le produit de la réaction du composé de formule (II) avec le composé de formule (III).

**15.** Procédé selon l'une des revendications 12 à 14, **caractérisé en ce que** n, dans le composé de formule (III), est plus grand que 1, de préférence 2 ou 3, et **en ce qu'**on fait réagir 1 mole de ce composé avec moins de n mole(s) du composé de formule (III), n ayant la même signification que dans la formule (II), afin d'obtenir un mélange avec un composé de formule (I), dans lequel n est plus grand que 1 et désigne de préférence 2 ou 3, et avec un composé de formule (II) dans lequel n désigne 1 et où le groupe {B} contient au moins un radical $[Y\text{-}(CHR^5)_mC(O)X\text{-}]$.

**16.** Homopolymères d'un composé de formule (I)

$$[(R^1)(R^2)P(O)\text{-}(CHR^3)\text{-}(CHR^4)\text{-}(CHR^5)_m\text{-}C(O)X\text{-}]_n\{B\} \qquad I$$

dans laquelle les radicaux et indices ont la signification suivants :

X est O, NH, $NR^6$ ou S,
$R^1$, $R^2$ sont, indépendamment l'un de l'autre, l'hydrogène ou un groupe alkyle, alcényle, aryle, alkylaryle ou arylalkyle éventuellement substitué ou un groupe alcoxy, aryloxy, alkylaryloxy, arylalkyloxy ou hydroxy éventuellement substitué,
$R^3$, $R^4$, $R^5$ et $R^6$ sont, indépendamment les uns des autres, l'hydrogène ou un groupe alkyle, alcényle, aryle, alkylaryle ou arylalkyle éventuellement substitué,
{B} est un radical à chaîne droite ou ramifiée ayant plus d'un groupe organiquement polymérisable et au moins 2 atomes de carbone et ne contenant pas d'atomes de silicium,
m est un nombre entier de 0 à 20,
n est un nombre entier de 1 à 20.

**17.** Homopolymères selon la revendication 16, dans lesquels les radicaux et indices indiqués dans la formule (I) ont la signification indiquée dans l'une des revendications 2 à 11.

**18.** Polymères mixtes de différents composés de formule (I) :

$$[(R^1)(R^2)P(O)\text{-}(CHR^3)\text{-}(CHR^4)\text{-}(CHR^5)_m\text{-}C(O)X\text{-}]_n\{B\} \qquad I$$

dans laquelle les radicaux et indices ont la signification suivants :

X est O, NH, $NR^6$ ou S,
$R^1$, $R^2$ sont, indépendamment l'un de l'autre, l'hydrogène ou un groupe alkyle, alcényle, aryle, alkylaryle ou arylalkyle éventuellement substitué ou un groupe alcoxy, aryloxy, alkylaryloxy, arylalkyloxy ou hydroxy éventuellement substitué,
$R^3$, $R^4$, $R^5$ et $R^6$ sont, indépendamment les uns des autres, l'hydrogène ou un groupe alkyle, alcényle, aryle, alkylaryle ou arylalkyle éventuellement substitué,
{B} est un radical à chaîne droite ou ramifiée ayant plus d'un groupe organiquement polymérisable et au moins 2 atomes de carbone et ne contenant pas d'atomes de silicium,
m est un nombre entier de 0 à 20,
n est un nombre entier de 1 à 20.

**19.** Polymères mixtes selon la revendication 18, dans lesquels les radicaux et indices indiqués dans la formule (I) ont la signification indiquée dans l'une des revendications 2 à 11.

**20.** Copolymérisats, formés en utilisant des motifs monomères de formule (I) ou des motifs polymères à blocs qui sont formés à partir de monomères de formule (I),

$$[(R^1)(R^2)P(O)\text{-}(CHR^3)\text{-}(CHR^4)\text{-}(CHR^5)_m\text{-}C(O)X\text{-}]_n\{B\} \qquad I$$

dans laquelle les radicaux et indices ont la signification suivants :

X est O, NH, $NR^6$ ou S,
$R^1$, $R^2$ sont, indépendamment l'un de l'autre, l'hydrogène ou un groupe alkyle, alcényle, aryle, alkylaryle ou

arylalkyle éventuellement substitué ou un groupe alcoxy, aryloxy, alkylaryloxy, arylalkyloxy ou hydroxy éventuellement substitué,
$R^3$, $R^4$ , $R^5$ et $R^6$ sont, indépendamment les uns des autres, l'hydrogène ou un groupe alkyle, alcényle, aryle, alkylaryle ou arylalkyle éventuellement substitué,
{B} est un radical à chaîne droite ou ramifiée ayant plus d'un groupe organiquement polymérisable et au moins 2 atomes de carbone et ne contenant pas d'atomes de silicium,
m est un nombre entier de 0 à 20,
n est un nombre entier de 1 à 20.

**21.** Copolymérisats selon la revendication 20, dans lesquels les radicaux et indices indiqués dans la formule (I) ont la signification indiquée dans l'une des revendications 2 à 11.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2711234 C3
- EP 0554890 A
- DE 19918974 A1
- DE 19746708 C2
- WO 0174826 A1
- US 3762865 A
- US 3839207 A
- WO 0058316 A
- DE 4416857 A1